# EUROPÄISCHE PATENTSCHRIFT

(11) **EP 2 198 791 B1**
(45) Veröffentlichungstag und Bekanntmachung des Hinweises auf die Patenterteilung: **09.05.2012**
(21) Anmeldenummer: 10158084.3
(22) Anmeldetag: 13.02.2004
(51) Int. Cl.: A61B 17/58, A61B 17/68

(54) **Knochenplatten-Fixiervorrichtung**
Fixation device for bone plaques
Elément de fixation pour plaque osseuse

(30) Priorität: 27.02.2003 DE 10310004
(43) Veröffentlichungstag der Anmeldung: 23.06.2010
(62) Teilanmeldung aus: 04710804.8
(73) Patentinhaber: Aesculap AG, 78532 Tuttlingen (DE)
(72) Erfinder: Pleil, Thomas, 78073, Bad Dürrheim (DE); Steinhilper, Klaus-Dieter, 78532, Tuttlingen (DE); Weißhaupt, Dieter, 78194, Immendingen (DE); Nesper, Markus, 78532, Tuttlingen (DE)
(74) Vertreter: Hoeger, Stellrecht & Partner Patentanwälte

(56) Entgegenhaltungen:
- WO-A1-2004/028384
- DE-C1- 19 832 797
- DE-U1- 29 919 090
- US-B1- 6 379 363

## Beschreibung

Die Erfindung betrifft eine Knochenplatten-Fixiervorrichtung gemäß dem Oberbegriff des Anspruchs 1.

Derartige Knochenplatten-Fixiervorrichtungen sind beispielsweise aus der DE 198 32 797 C1 bekannt. In der US 6,379,363 B1 und in der DE 299 19 090 U1 sind ähnliche Knochenplatten-Fixiervorrichtungen beschrieben, die jedoch keinen Vorsprung in Form eines Kopfes aufweisen, welcher an einem vom ersten Knochenanlageelement weg weisenden Ende des Verbindungsglieds angeordnet ist. Eine weitere Knochenplatten-Fixiervorrichtung ist in der WO 2004/028384 A1 beschrieben, bei welcher das zweite Knochenanlageelement mittels einer mit dem Verbindungselement verschraubbaren Rückhaltehülse in Richtung auf das erste Knochenanlageelement bewegbar ist.

Ein chirurgisches Instrument zum Anlegen einer Knochenplatten-Fixiervorrichtung, welche ein erstes Knochenanlageelement mit einem von diesem abstehenden stabförmigen, eine Längsrichtung definierenden Verbindungsglied und ein auf dem Verbindungsglied in Richtung auf das erste Knochenanlageelement verschiebbares zweites Knochenanlageelement aufweist, mit einem an dem zweiten Knochenanlageelement in einer Anlagestellung anlegbaren ersten Werkzeugelement und einem von dem ersten Werkzeugelement entfernbaren zweiten Werkzeugelement, mit einer Transportvorrichtung zum schrittweisen Transportieren des Verbindungsglieds mit dem zweiten Werkzeugelement in mehreren Transportschritten in einer proximalen Richtung weg von dem in der Anlagestellung am zweiten Knochenelement anliegenden ersten Werkzeugelement ist beispielsweise aus der DE 197 00 474 C2 bekannt. Mit einem von zwei Spannbacken gebildeten zweiten Werkzeugelement kann das stabförmige Verbindungsglied in einer Klemmstellung geklemmt und in der Klemmstellung relativ zum zweiten Knochenanlageelement bewegt werden. Ein Nachfassen des Verbindungsglieds mit den Spannbacken ist in der oben beschriebenen Art und Weise möglich.

Mit dem bekannten Instrument ist jedoch ein definierter Transport des Verbindungsglieds relativ zum zweiten Knochenanlageelement nicht eindeutig gewährleistet. Ferner ist es schwierig, ein glattes Verbindungsglied sicher zu greifen. Bei strukturierten Verbindungsgliedern ergibt sich das Problem, dass sich eine Struktur des Verbindungsglieds in die Spannbacken eingraben und zu einer Beschädigung derselben führen kann. In jedem Fall besteht die Gefahr, bei hohen auf das zweite Werkzeugelement wirkenden Zugkräften, dass die Spannbacken am Verbindungsglied abrutschen können. Ferner lässt sich das Instrument nur schwer reinigen, wenn die Spannbacken Beschädigungen aufgrund scharfkantiger Strukturen der Verbindungsglieder aufweisen.

Es ist daher Aufgabe der vorliegenden Erfindung, eine Knochenplatten-Fixiervorrichtung der eingangs beschriebenen Art so zu verbessern, dass die Knochenanlageelemente der Fixiervorrichtung auf einfache Weise relativ zueinander verschoben werden können.

Diese Aufgabe wird bei einer Knochenplatten-Fixiervorrichtung der eingangs beschriebenen Art erfindungsgemäß dadurch gelöst, dass der Vorsprung eine ringförmige Einschnürung aufweist.

Weitere bevorzugte Ausführungsformen der Knochenplatten-Fixiervorrichtung sind Gegenstand der Unteransprüche.

Die nachfolgende Beschreibung dient im Zusammenhang mit der Zeichnung der näheren Erläuterung. Es zeigen:
- Figur 1:: eine Spannzange mit Spannbacken in einer distalen Lösestellung;
- Figur 2:: das Instrument aus Figur 1 mit den Spannbacken in einer distalen Eingriffsposition;
- Figur 3:: das Instrument aus Figur 1 mit den Spannbacken in einer proxima- len Zugstellung;
- Figur 4:: das Instrument aus Figur 3 bei wirkender Zugkraftbegrenzung;
- Figur 5:: eine mögliche erste Eingriffsposition eines Vorsprungs eines Verbin- dungsglieds an Zähnen der Spannbacken; und
- Figur 6:: eine zweite mögliche Eingriffsposition des Vorsprungs an den Zäh- nen der Spannbacken.

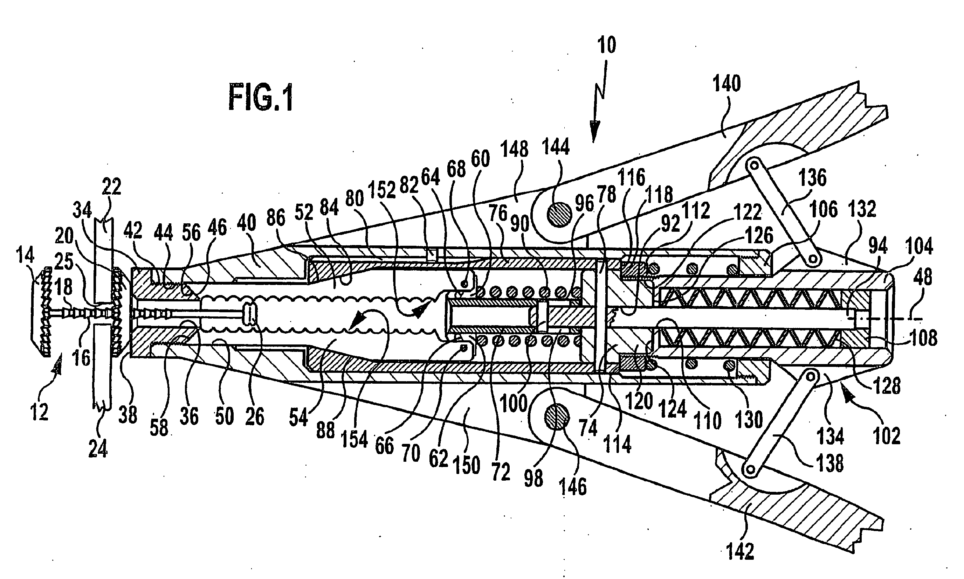

In den Figuren 1 bis 4 ist ein Instrument in Form einer chirurgischen Spannzange dargestellt. Die Spannzange 10 dient zum Anlegen eines erfindungsgemäßen, nietartigen Fixierungselements 12, welches ein erstes Anlageelement 14 mit einem von diesem abstehenden, mit Rückhaltevorsprüngen 16 versehenen langgestreckten Schaft 18 sowie ein zweites Anlageelement 20 umfasst, welches relativ zum ersten Anlageelement 14 auf dem Schaft 18 in Richtung auf das erste Anlageelement 14 hin verschiebbar ist. Eine Verschiebung des zweiten Anlageelements 20 relativ zum ersten Anlageelement 14 von diesem weg ist aufgrund der in diese Richtung wirkenden Rückhaltevorsprünge 16 nicht möglich. Zwischen den Anlageelementen 14 und 20 können zwei getrennte Knochenteile 22 und 24, welche beispielsweise Teile eines menschlichen Schädelknochen bilden, miteinander fixiert werden, indem die beiden Anlageelemente 14 und 20 die Knochenteile 22 und 24 beidseitig zwischen sich einklemmen.

An einem vom ersten Anlageelement 14 weg weisenden Ende des Schafts 18 ist ein ringförmiger Vorsprung 26 angeordnet, welcher eine ringförmige Einschnürung 28 aufweist. Der Vorsprung 26 ist auf diese Weise quasi mit einer Verzahnung umfassend zwei Zähne 30 und 32 versehen.

Mittels der Spannzange 10 ist eine Relativbewegung zwischen den beiden Anlageelementen 14 und 20 realisierbar. Hierfür umfasst die Spannzange 10 ein erstes Werkzeugelement in Form einer mit einer Längsbohrung 36 versehenen Einschraubhülse 34, die eine ringförmige, in distaler Richtung weisende Anlagefläche 38 zum Anlegen am zweiten Anlageelement 20 aufweist. Die Längsbohrung 36 ist so bemessen, dass der Schaft 18 mit dem Vorsprung 26 durch die Einschraubhülse 34 hindurchgeführt werden kann.

Die Einschraubhülse 34 ist mit einem Außengewindeabschnitt 42 versehen, welcher zu einem Innengewindeabschnitt 44 an einem distalen Ende eines Grundkörpers 40 der Spannzange 10 korrespondiert. An ihrem proximalen Ende weist die Einschraubhülse 34 eine Kegelmantelfläche 46 auf, die in proximaler Richtung weist. Eine Spitze eines von der Kegelmantelfläche 46 definierten Kegels würde auf einer Längsachse 48 der Spannzange 10 liegen, welche gleichzeitig eine Symmetrieachse der Spannzange 10 und des Fixierungselementes 12 bildet.

Der Grundkörper 40 ist in Form einer langgestreckten Hülse ausgebildet und weist an die Kegelmantelfläche 46 angrenzend einen ringförmigen Anlageabschnitt 50 für zwei symmetrisch zur Längsachse 48 angeordnete langgestreckte Spannbacken 52 und 54 auf. Distalseitig sind die Spannbacken 52 und 54 jeweils mit einer zur Kegelmantelfläche 46 korrespondierenden schrägen Aufgleitfläche 56 beziehungsweise 58 versehen. Proximalseitig sind freie Enden der Spannbacken 52 und 54 an Lagerlappen 60 und 62 sowohl schwenkbar als auch verschiebbar gelagert, und zwar indem ein drehfest an den Spannbacken 52 beziehungsweise 54 quer zur Längsachse 48 orientierter Stift 68 beziehungsweise 70 einen an den Lagerlappen 60 beziehungsweise 62 schräg von der Längsachse 48 in proximaler Richtung weisenden Schlitz 64 beziehungsweise 66 durchsetzt. Die Lagerlappen 60 und 62 sind distalseitig radial abstehend an einer Zughülse 72 angeordnet, welche proximalseitig mit einem rotationssymmetrisch zur Längsachse 48 geformten Lagerzapfen 74 verbunden ist. Der Lagerzapfen 74 ist distalseitig in ein proximales Ende einer Klemmhülse 76 mittels eines quer zur Längsachse 48 sowohl den Lagerzapfen 74 als auch die Klemmhülse 76 durchsetzenden Bolzens 78 drehfest und auch axial verschieblich gesichert.

Die Klemmhülse 76 ist im Grundkörper 40 axial verschieblich gelagert und gegen ein Verdrehen relativ zum Grundkörper 40 gesichert durch eine sich außen von einem proximalen Ende der Klemmhülse 76 weg erstreckenden Längsnut 80, in welche ein innen von der Klemmhülse 76 in Richtung auf die Längsachse 48 hin weisend abstehender Sicherungszapfen 82 eintaucht. Die Klemmhülse 76 weist distalseitig einen sich verringernden Innendurchmesser auf, wodurch eine Umlenkfläche 84 gebildet wird, die schräg in proximaler Richtung auf die Längsachse 48 hin weist. Die Spannbacken 52 und 54 weisen zur Umlenkfläche 84 korrespondierend geneigte Gleitflächen 86 beziehungsweise 88 auf, die in einer in Figur 1 dargestellten Ausgangsstellung im wesentlichen vollständig an der Umlenkfläche 84 anliegen.

Eine die Zughülse 72 umgebende Spiralfeder 90 stützt sich einerseits an den Lagerlappen 60 und 62, andererseits am Lagerzapfen 74 ab. Die Spiralfeder 90 drückt somit die Spannbacken 52 und 54 in distaler Richtung mit ihren Gleitflächen 86 und 88 gegen die Umlenkfläche 84 sowie die Aufgleitflächen 56 und 58 gegen die Kegelmantelfläche 46.

Der Lagerzapfen 74 weist eine zentrale Bohrung 92 auf, in welcher ein zylindrischer langgestreckter Zugbolzen 94 eingesetzt und mittels des Bolzens 78 drehfest und axial unverschieblich am Lagerzapfen 74 gehalten ist. Distalseitig ist der Zugbolzen 94 in der Zughülse 72 verschiebbar gelagert, welche zwei sich parallel zur Längsachse 48 erstreckende Führungsschlitze 96 und 98 aufweist, in welche ein quer zur Längsachse 48 den Zugbolzen 94 durchsetzender Führungsstift 100 eintaucht und auf diese Weise die Zughülse 72 axial verschieblich und gegen eine Verdrehung gesichert am Zugbolzen 94 hält.

Proximalseitig ist der Zugbolzen 94 mit einer insgesamt mit dem Bezugszeichen 102 versehenen Zugkraftbegrenzung 102 verbunden. Diese umfasst eine Lagerhülse 104, welche längsverschieblich an einem in ein proximales Ende des Grundkörpers 40 eingeschraubten Lagerring 106 axial verschieblich geführt ist. Die Lagerhülse 104 führt in ihrem Inneren einen ringförmigen Kopf 108, welcher drehfest mit einem proximalen Ende des Zugbolzens 94 verbunden ist. Distalseitig wird der Zugbolzen 94 an einer zentralen axialen Hülsenbohrung 110 axial verschieblich geführt.

Auf ein distales Ende der Lagerhülse 104 ist außen ein Anschlagring 112 aufgeschraubt, welcher eine in distaler Richtung weisende Anschlagfläche 114 bildet. An der Anschlagfläche 114 liegt in der in Figur 1 dargestellten Grundstellung ein distales Ende 116 der Klemmhülse 76 sowie ein Ringvorsprung 118 des Lagerzapfens 74 an. Ein gegenüber dem Ringvorsprung 118 im Durchmesser verringerter Zapfenabschnitt 120 taucht in eine korrespondierende zylindrische Ausnehmung 122 der Lagerhülse 104 ein, welcher in distaler Richtung offen ist. Ein proximales Ende 126 des Lagerzapfens 74 stößt an einem Boden 124 der Ausnehmung 122 an, welche von der Hülsenbohrung 110 durchsetzt ist.

Den Zugbolzen 94 umgebend ist in der Lagerhülse 104 ein Tellerfederblock 128 angeordnet, der sich einerseits am Boden 124 und andererseits am Kopf 108 abstützt und so den Lagerzapfen 74 unter Vorspannung in der Ausnehmung 122 hält. Die Lagerhülse an ihrem distalseitigen Ende umgebend ist eine weitere Spiralfeder 130 innerhalb des Grundkörpers 40 angeordnet, die sich einerseits am Anschlagring 112 und andererseits am Lagerring 106 abstützt. Sie drückt damit insgesamt die Lagerhülse 104 in distaler Richtung.

Proximalseitig sind an der Lagerhülse 104 radial abstehend symmetrisch zwei Lagerböcke 132 und 134 angeordnet, an denen jeweils ein stabförmiger Lenker 136 beziehungsweise 138 schwenkbar gelagert ist. Die Lenker 136 und 138 sind ferner mit jeweils einem Schwenkgriff 140 beziehungsweise 142 schwenkbar verbunden. Die Schwenkgriffe 140 und 142 sind mittels zweier quer zur Längsachse 48 orientierter Gelenkbolzen 144 beziehungsweise 146 an radial von dem Grundkörper 40 abstehenden Lagerlappen 148 und 150 schwenkbar gehalten.

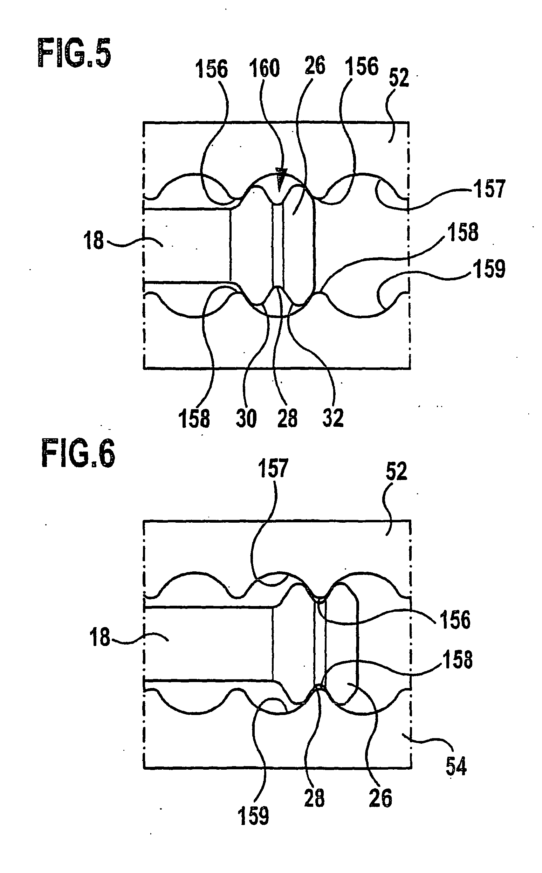

Die Spannbacken 52 und 54 sind jeweils mit einer Verzahnung 152 beziehungsweise 154 versehen, welche jeweils eine Vielzahl von in Richtung auf die Längsachse 58 hin weisenden Zähnen 156 und 158 aufweisen. Jeweils zwischen zwei Zähnen 156 und 158 sind Aufnahmen 157 beziehungsweise 159 bildende Vertiefungen ausgebildet. Die Zähne 156 und 158 sind allesamt verrundet. Ein Abstand der Zähne 156 und 158 voneinander ist so gewählt, dass der Vorsprung 26 als Ganzes zwischen zwei Zähne 156 und 158 einführbar ist. Eine solche Eingriffsposition ist in Figur 5 dargestellt.

Die Form einer Spitze der Zähne 156 und 158 entspricht jedoch auch im wesentlichen der Form der Einschnürung 28 des Vorsprungs 26 am Schaft 18, so dass jeweils ein Zahn 156 und 158 der Spannbacken 52 und 54 in die Einschnürung 28 eintauchen kann. Eine solche Eingriffsposition ist in Figur 6 dargestellt. Die Verzahnungen 152 und 154 sind so gewählt, dass die Zähne 30 und 32 des Vorsprungs 26 halb so weit voneinander entfernt sind wie jeweils zwei Zähne 156 beziehungsweise 158. Dadurch entspricht eine Teilung einer Verzahnung 160 des Vorsprungs 26 zweimal der Teilung der Verzahnungen 152 und 154. Damit lassen sich Eingriffspositionen definieren, die dem halben Abstand der Teilung der Verzahnungen 152 und 154 entsprechen. Zwei derartige, in einem solchen Abstand voneinander getrennte Eingriffspositionen sind in den Figuren 5 und 6 dargestellt.

In Verbindung mit den Figuren 1 bis 4 wird nachfolgend näher erläutert, wie mittels der Spannzange 10 das zweite Anlageelement 20 relativ zum Schaft 18 in Richtung auf das erste Anlageelement 14 hin verschoben werden kann.

Zunächst werden die beiden Anlageelemente 14 und 20 beidseitig der zwei miteinander zu verbindenden Knochenteile 22 und 24 an diese angelegt und der Schaft 14 durch einen Knochenspalt 25 hindurchgeführt. Der Schaft 18 mit dem Vorsprung 26 wird durch die Einschraubhülse 34 hindurchgesteckt. Die Einschraubhülse 34 wird an das zweite Anlageelement 20 angelegt. Diese Grundstellung ist in Figur 1 dargestellt.

Durch Verschwenken der Schwenkgriffe 140 und 142 in Richtung auf die Längsachse 48 hin, wird die Lagerhülse 104 in proximaler Richtung gezogen und drückt die Spiralfeder 130 zusammen. Solange die von den Schwenkgriffen 140 und 142 ausgeübte Kraft kleiner als die von dem Tellerfederblock 128 ausgeübte Kraft ist, wird der Lagerzapfen 74 in der Ausnehmung 122 der Lagerhülse 104 gehalten. Zusammen mit dem Lagerzapfen 74 wird die Klemmhülse 76 in proximaler Richtung gezogen, wodurch die Gleitflächen 86 und 88 der Spannbacken 52 und 54 an der Umlenkfläche 84 der Klemmhülse 76 aufgleiten. Die Umlenkfläche 84 wirkt somit als Umlenkglied, mit welchem eine in Richtung der Längsachse 48 wirkende Zugkraft in eine Schubkraft in Richtung auf die Längsachse 48 hin umgelenkt wird. Die Spannbacken 52 und 54 werden zwangsgeführt in Richtung auf die Längsachse 48 hin bewegt, wobei eine Führung einerseits durch die an der Kegelmantelfläche 46 anliegenden Aufgleitflächen 56 und 58 realisiert wird, andererseits mittels der in den Schlitzen 64 und 66 geführten Stifte 68 und 70.

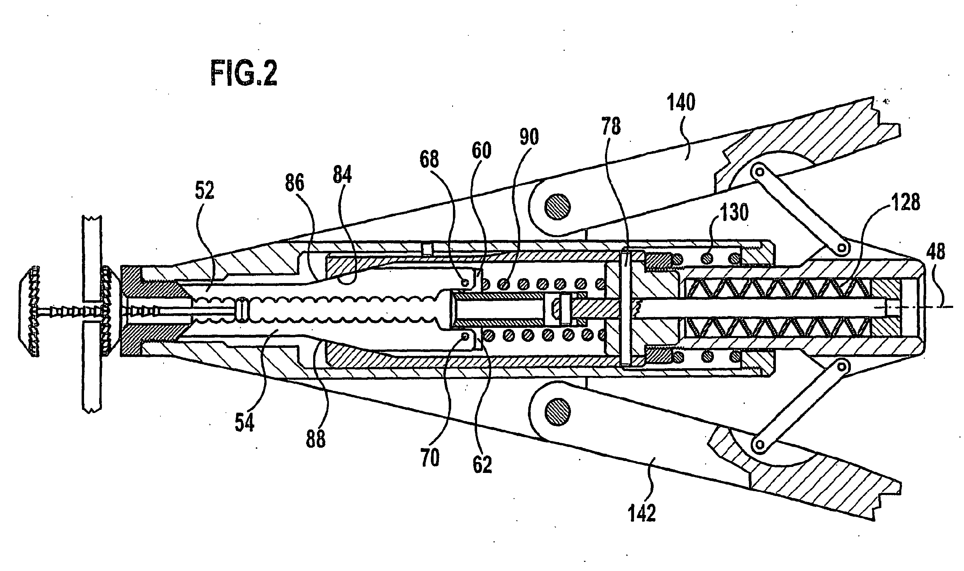

Die Spannbacken 52 und 54 können so weit in Richtung auf die Längsachse 48 hin bewegt werden, bis die Verzahnungen 152 und 154 mit dem Vorsprung 26 in Eingriff kommen. Hierzu gibt es zwei Eingriffspositionen, die im Zusammenhang mit den Figuren 5 und 6 bereits näher erläutert wurden. Figur 2 zeigt die Eingriffsposition der Spannbacken 52 und 54 am Vorsprung 26 in einer distalen Stellung derselben. Figur 5 entspricht einem vergrößerten Ausschnitt der Figur 2.

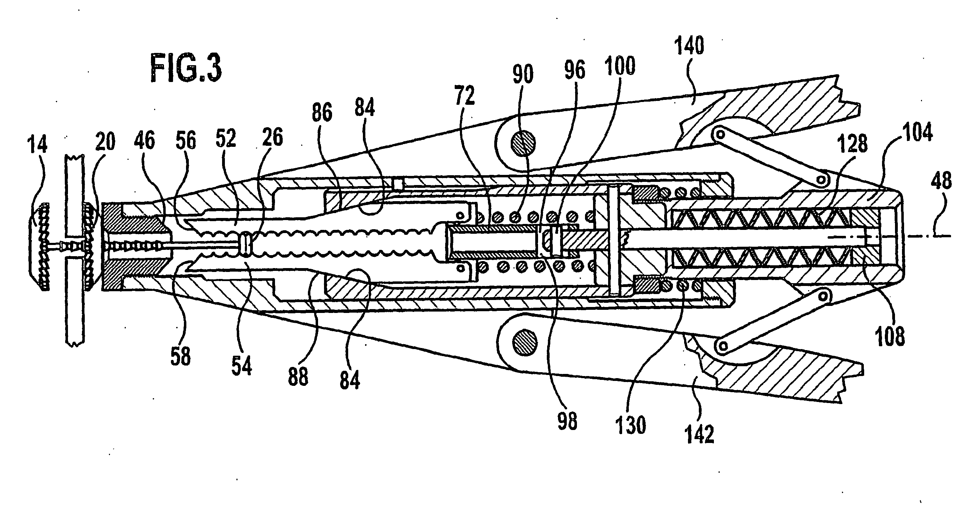

Werden die Schwenkgriffe 140 und 142 weiter in Richtung auf die Längsachse 48 hin verschwenkt, werden die Spannbacken 52 und 54 in proximaler Richtung mitgenommen. Die Kraft der Spiralfeder 90 reicht nicht aus, um die Spannbacken 52 und 54 weiter in distaler Richtung vorzuspannen. In Figur 3 ist eine Stellung der Spannzange 10 gezeigt, bei der der Vorsprung 26 relativ vom zweiten Anlageelement 20 weg bewegt wurde, so dass das zweite Anlageelement 20 bereits eine in Richtung auf das erste Anlageelement 14 hin veränderte Position einnimmt.

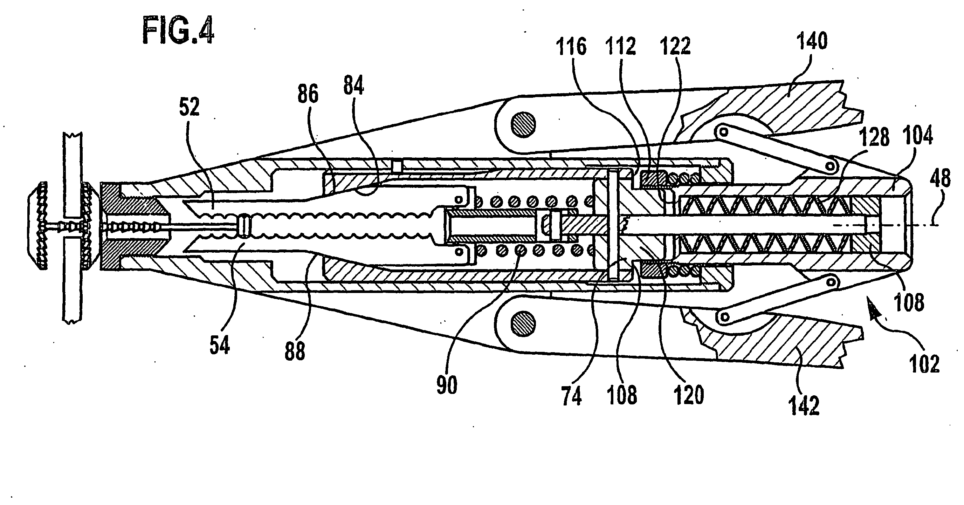

Werden die Schwenkgriffe 140 und 142 noch weiter in Richtung auf die Längsachse 48 hin verschwenkt, so beginnt die Zugkraftbegrenzung 102 zu wirken. Die auf die Lagerhülse 104 ausgeübte Zugkraft übersteigt nunmehr die vom Tellerfederblock 128 ausgeübte Kraft, wodurch der Tellerfederblock 128 komprimiert wird. Eine axiale Position der Klemmhülse 76 relativ zum Grundkörper 40 bleibt dadurch praktisch konstant. Dagegen wird die Spiralfeder 130 ebenso wie der Tellerfederblock 128 weiter zusammengedrückt. Diese Stellung ist in Figur 4 dargestellt.

Zum Nachfassen des Vorsprungs 26 mit den Spannbacken 52 und 54 werden die Schwenkgriffe 140 und 142 wieder von der Längsachse 48 weg verschwenkt. Dies kann beispielsweise mittels einer nicht dargestellten Blattfeder automatisch erfolgen. Durch entsprechende Auswahl der Spiralfedern 90 und 130 ermöglicht es die Anordnung der Spannzange 10, dass in der in Figur 3 dargestellten Zugposition beim Rückverschwenken der Schwenkgriffe 140 und 142 von der Längsachse 48 weg zunächst die Spannbacken 52 und 54 radial von der Längsachse 48 und vom Vorsprung 26 weg bewegt werden, wenn der Zug auf die Lagerhülse 104 reduziert wird. Damit geben die Spannbacken 52 und 54 den Vorsprung 26 am Schaft 18 frei. Ein weiteres Verschwenken der Schwenkgriffe 140 und 142 zurück in die in Figur 1 dargestellte Grundstellung führt dazu, dass die Spannbacken 52 und 54 in distaler Richtung bewegt werden, dabei jedoch nicht in Eingriff mit dem Vorsprung 26 stehen. Sobald die Aufgleitflächen 56 und 58 wieder an der Kegelmantelfläche 46 anliegen, kann der Vorsprung 26 in einem weiteren Transportschritt weiter in proximaler Richtung bewegt werden.

Insgesamt werden so viele Transportschritte in der oben beschriebenen Weise durchgeführt, bis die beiden Knochenteile 22 und 24 klemmend zwischen den beiden Anlageelementen 14 und 20 gehalten werden.

## Patentansprüche

1. Knochenplatten-Fixiervorrichtung (12), welche ein erstes Knochenanlageelement (14) mit einem von diesem abstehenden stabförmigen, eine Längsrichtung (48) definierenden Verbindungsglied (18), und ein auf dem Verbindungsglied (18) in Richtung auf das erste Knochenanlageelement (14) verschiebbares zweites Knochenanlageelement (20) aufweist, welches Verbindungsglied (18) mit Rückhaltevorsprüngen (16) versehen ist, wodurch eine Verschiebung des zweiten Knochenanlageelements (20) relativ zum ersten Knochenanlageelement (14) von diesem weg aufgrund der in diese Richtung wirkenden Rückhaltevorsprünge (16) nicht möglich ist, wobei das Verbindungsglied (18) einen abstehenden Vorsprung (26) in Form eines im Durchmesser über das Verbindungsglied (18) vorspringenden Kopfs aufweist und wobei der Vorsprung (26) an einem vom ersten Knochenanlageelement (14) weg weisenden Ende des Verbindungsglieds (18) angeordnet ist, **dadurch gekennzeichnet, dass** der Vorsprung (26) eine ringförmige Einschnürung (28) aufweist.

2. Knochenplatten-Fixiervorrichtung nach einem der voranstehenden Ansprüche, **dadurch gekennzeichnet, dass** der Vorsprung (26) ringförmig ausgebildet ist.

3. Knochenplatten-Fixiervorrichtung nach einem der voranstehenden Ansprüche, **dadurch gekennzeichnet, dass** der Vorsprung (26) kantenfrei ausgebildet ist.

4. Knochenplatten-Fixiervorrichtung nach einem der voranstehenden Ansprüche, **dadurch gekennzeichnet, dass** der Vorsprung (26) mindestens teilweise mit einer Aufnahme (157, 159) eines chirurgischen Instruments (10) zum Anlegen einer Knochenplatten-Fixiervorrichtung (12) in einer Eingriffsposition in Eingriff bringbar und in dieser in Längsrichtung (48) unbeweglich haltbar ist.

5. Knochenplatten-Fixiervorrichtung nach einem der voranstehenden Ansprüche, **dadurch gekennzeichnet, dass** das Verbindungsglied (18) in Form eines langgestreckten Schafts ausgebildet ist.

6. Knochenplatten-Fixiervorrichtung nach einem der voranstehenden Ansprüche, **dadurch gekennzeichnet, dass** die Knochenplatten-Fixiervorrichtung (12) nietartig ausgebildet ist.

## Claims

1. Bone plate fixing device (12) comprising a first bone contacting element (14) with a rod-shaped connecting member (18) projecting from the first bone contacting element and defining a longitudinal direction (48), and a second bone contacting element (20) displaceable on the connecting member (18) in a direction towards the first bone contacting element (14), said connecting member (18) having retaining projections (16), whereby a displacement of the second bone contacting element (20) relative to the first bone contacting element (14) away from the first bone contacting element is not possible owing to the retaining projections (16) acting in this direction, wherein the connecting member (18) has a protruding projection (26) in the form of a head protruding in its diameter beyond the connecting member (18), and wherein the projection (26) is arranged at an end of the connecting member (18) that points away from the first bone contacting element (14), **characterized in that** the projection (26) has a ring-shaped constriction (28).

2. Bone plate fixing device in accordance with any one of the preceding claims, **characterized in that** the projection (26) is ring-shaped.

3. Bone plate fixing device in accordance with any one of the preceding claims, **characterized in that** the projection (26) is of edge-free design.

4. Bone plate fixing device in accordance with any one of the preceding claims, **characterized in that** the projection (26) is at least partly engageable with a receptacle (157, 159) of a surgical instrument (10) for applying a bone plate fixing device (12) in an engagement position and is holdable therein immovably in longitudinal direction (48).

5. Bone plate fixing device in accordance with any one of the preceding claims, **characterized in that** the connecting member (18) is in the form of an elongate shaft.

6. Bone plate fixing device in accordance with any one of the preceding claims, **characterized in that** the bone plate fixing device (12) is of rivet-like design.

## Revendications

1. Dispositif de fixation de plaque d'ostéosynthèse (12), qui comprend un premier élément d'appui sur l'os (14) dont fait saillie un organe de liaison (18) en forme de tige définissant une direction longitudinale (48), et un deuxième élément d'appui sur l'os (20) pouvant coulisser sur l'organe de liaison (18) en direction du premier élément d'appui sur l'os (14), cet organe de liaison (18) étant muni de protubérances de retenue (16) rendant impossible un coulissement du deuxième élément d'appui sur l'os (20) par rapport au premier élément d'appui sur l'os (14) en s'éloignant de celui-ci, en raison des protubérances de retenue (16) agissant dans cette direction, l'organe de liaison (18) comportant en outre une protubérance (26) en saillie, qui se présente sous la forme d'une tête dépassant en diamètre de l'organe de liaison (18), et la protubérance (26) étant agencée à une extrémité de l'organe de liaison (18), qui est opposée à celle où se situe le premier élément d'appui sur l'os (14),
**caractérisé en ce que** la protubérance (26) présente un rétrécissement (28) de forme annulaire.

2. Dispositif de fixation de plaque d'ostéosynthèse selon la revendication 1, **caractérisé en ce que** la protubérance (26) est réalisée sous une forme annulaire.

3. Dispositif de fixation de plaque d'ostéosynthèse selon l'une des revendications précédentes, **caractérisé en ce que** la protubérance (26) est réalisée sans arêtes.

4. Dispositif de fixation de plaque d'ostéosynthèse selon l'une des revendications précédentes, **caractérisé en ce que** la protubérance (26) peut être amenée au moins partiellement en prise avec un logement de réception (157, 159) d'un instrument chirurgical (10) pour l'application d'un dispositif de fixation de plaque d'ostéosynthèse (12), dans une position de prise dans laquelle elle est alors maintenue de manière non mobile dans la direction longitudinale (48).

5. Dispositif de fixation de plaque d'ostéosynthèse selon l'une des revendications précédentes, **caractérisé en ce que** l'organe de liaison (18) est réalisé sous la forme d'un corps allongé en forme de tige.

6. Dispositif de fixation de plaque d'ostéosynthèse selon l'une des revendications précédentes, **caractérisé en ce que** le dispositif de fixation de plaque d'ostéosynthèse (12) est d'une configuration du type rivet.
